# EUROPEAN PATENT SPECIFICATION

(11) **EP 0 980 383 B1**
(45) Date of publication and mention of the grant of the patent: **02.01.2003**
(21) Application number: 98918801.6
(22) Date of filing: 28.04.1998
(51) Int. Cl.: C07J 31/00, A61K 31/565, C07J 1/00

(54) **PHARMACEUTICALLY ACCEPTABLE SALTS OF 3-HYDROXY-ESTR-5(10)-EN-17-ONE 3-SULPHATE ACTIVE AS ESTROGENS**
PHARMAZEUTISCH VERTRÄGLICHE SALZE VON 3-HYDROXY-ESTR-5(10)-EN-17-ON-3-SULFAT DIE ALS ÖSTROGENE AKTIV SIND
SELS PHARMACEUTIQUEMENT ACCEPTABLES DE 3-HYDROXY-ESTR-5(10)-EN-17-ONE 3-SULFATE A ACTIVITE D'OESTROGENES

(30) Priority: 02.05.1997 US 850568
(43) Date of publication of application: 23.02.2000
(73) Proprietor: Wyeth, Madison, New Jersey 07940-0874 (US)
(72) Inventor: KAGAN, Michael, Z., Plainsboro, NJ 08536 (US); KONG, Fangming, Riverdale NJ 07675 (US); McDONALD, Leonard, Alexander, Mountainside, NJ 07092 (US); RAVEENDRANATH, Panolil, Monroe, NY 10950 (US); SHAH, Syed, Muzafar, East Hanover, NJ 07936 (US); ZELDIS, Joseph, New City, NY 10956 (US)
(74) Representative: Talbott, Dawn Jacqueline
(86) International application number: US9808481
(87) International publication number: WO98050413

(56) References cited:
- CHEMICAL ABSTRACTS, vol. 114, no. 25, 24 June 1991 Columbus, Ohio, US; abstract no. 240851, LEE C M H ET AL: "Conversion of 5(10)-estrene-3.beta.,17.beta.-diol to 19-nor-4-ene-3-ketosteroids by luteal cells in vitro: possible involvement of the 3.beta.-hydroxysteroid dehydrogenase/isomerase" page 121; column 1; XP002072926 & J. ENDOCRINOL., vol. 129, no. 2, 1991, pages 233-243,
- TOWNSLEY J D ET AL: "Mechanism of estrogen biosynthesis. III. Stereochemistry of aromatization of C19 and C18 steroids" BIOCHEMISTRY, vol. 7, no. 1, January 1968, pages 33-40, XP002072922 EASTON, PA US
- DUMASIA M C ET AL: "Biotransformation of 5(10)-estrene-3.alpha.,17.beta.-diol by equine testicular preparations in vitro" BIOCHEM. SOC. TRANS., vol. 17, no. 6, 1989, pages 1019-1020, XP002072923
- D. E. MARSHALL ET AL: "Studies into Aromatase Activity Associated with Fetal Allantochorionic and Maternal Endometrial Tissues of Equine Placenta. Identification of Metabolites by Gas Chromatography Mass Spectrometry" THE JOURNAL OF STEROID BIOCHEMISTRY AND MOLECULAR BIOLOGY, vol. 59, no. 3/4, November 1996, pages 281-296, XP002072924
- W. M. GARRETT ET AL: "Androgen Metabolism by Porcine Granulosa Cells during the Process of Luteinization in Vitro: Identification of 19-Oic-Androtenedione as a Major Metabolite and Possible Precursor for the Formation of C18 Neutral Steroids" ENDOCRINOLOGY, vol. 129, no. 6, 1991, pages 2941-2950, XP002072925

## Description

The use of naturally occurring estrogenic compositions of substantial purity and low toxicity such as PREMARIN™ (conjugated equine estrogens) has become a preferred medical treatment for alleviating the symptoms of menopausal syndrome, osteoporosis/osteopenia in estrogen deficient women and in other hormone related disorders. The estrogenic components of the naturally occurring estrogenic compositions have been generally identified as sulfate esters of estrone, equilin, equilenin, 17-β-estradiol, dihydroequilenin and 17-β-dihydroequilenin (U.S. Patent 2,834,712). The estrogenic compositions are usually buffered or stabilized with alkali metal salts of organic or inorganic acids at a substantially neutral pH of about 6.5 to 7.5. Urea has also been used as a stabilizer (U.S. 3,608,077). The incorporation of antioxidants to stabilize synthetic conjugated estrogens and the failure of pH control with tris(hydroxymethyl)aminomethane (TRIS) to prevent hydrolysis is discussed in U.S. 4,154,820.

One of the compounds described herein, 3β-hydroxy-5(10)-estrene-17-one 3-sulfate ester sodium salt is a minor component of PREMAPIN™ (conjugated equine estrogens).

### DESCRIPTION OF THE INVENTION

In accordance with this invention, there is provided a pharmaceutically acceptable salt of 3β-hydroxy-5(10)-estrene-17-one 3-sulfate ester, and a pharmaceutically acceptable salt of 3α-hydroxy-5(10)-estrene-17-one 3-sulfate ester. These are collectively referred to as the compounds of this invention. The structure of 3β-hydroxy-5(10)-estrene-17-one 3-sulfate ester sodium salt is shown in Scheme I as compound (**9**).

Pharmaceutically acceptable salts of 3β-hydroxy-5(10)-estrene-17-one 3-sulfate ester and 3α-hydroxy-5(10)-estrene-17-one 3-sulfate ester include, but are not limited to, the alkali metal salts, alkaline earth metal salts, ammonium salts, alkylammonium salts containing 1-6 carbon atoms or dialkylammonium salts containing 1-6 carbon atoms in each alkyl group, trialkylammonium salts containing 1-6 carbon atoms in each alkyl group and tetraalkylammonium salts containing 1-6 carbon atoms in each alkyl group.

Alkali metal salts include sodium and potassium salts, particularly preferred are sodium salts. Alkaline earth metal salts include calcium and magnesium salts. Suitable alkyl groups include methyl, ethyl, propyl, butyl, pentyl and hexyl, preferred alkyl groups being methyl and ethyl. Where more than one alkyl group is present the groups may be the same or different. Preferred trialkylammonium salts are trimethylammonium salts and triethylammonium salts.

As used in accordance with this invention, treating covers treatment of an existing condition, ameliorating the condition, or providing palliation of the condition and inhibiting includes inhibiting or preventing the progress or development of the condition.

The present invention further provides compositions comprising a pharmaceutically acceptable salt of 3β-hydroxy-5(10)-estrene-17-one 3-sulfate ester, a pharmaceutically acceptable salt of 3α-hydroxy-5(10)-estrene-17-one 3-sulfate ester or a mixture thereof. In particular it provides compositions comprising at least 1% of 3β-hydroxy-5(10)-estrene-17-one 3-sulfate ester, a pharmaceutically acceptable salt of 3α-hydroxy-5(10)-estrene-17-one 3-sulfate ester or a mixture thereof. One aspect of the present invention provides compositions wherein the only estrogenic agent is 3β-hydroxy-5(10)-estrene-17-one 3-sulfate ester, a pharmaceutically acceptable salt of 3α-hydroxy-5(10)-estrene-17-one 3-sulfate ester or a mixture thereof. Embodiments of the present invention include compositions wherein the only active compound is 3β-hydroxy-5(10)-estrene-17-one 3-sulfate ester, a pharmaceutically acceptable salt of 3α-hydroxy-5(10)-estrene-17-one 3-sulfate ester or a mixture thereof. In these embodiments other excipients and carriers may be included but no further active materials are included.

The present invention also provides a compound as described above for use as a medicament.

The present invention also provides the use of such a compound in the preparation of a medicament for:
a) inhibiting or treating free radical induced disease states,
b) inhibiting endogenous free radical involvement in disease development of cancers, central nervous system disorders, dementias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures,
c) providing estrogen replacement therapy or treating estrogen deficiency,
d) treating vasomotor symptoms related to estrogen deficiency,
e) treating or inhibiting osteoporosis or
f) treating or inhibiting atherosclerosis in a mammal.

The present invention also provides a process for the preparation of a pharmaceutically acceptable salt of 3α-hydroxy-5(10)-estrene-17-one 3-sulfate ester or a pharmaceutically acceptable salt of 3β-hydroxy-5(10)-estrene-17-one 3-sulfate ester which comprises:
a) converting 3α-hydroxy-5(10)-estrene-17-one 3-sulfate ester or 3β-hydroxy-5(10)-estrene-17-one 3-sulfate ester to a pharmaceutically acceptable salt or
b) converting a pharmaceutically acceptable salt of 3α-hydroxy-5(10)-estrene-17-one 3-sulfate ester or 3β-hydroxy-5(10)-estrene-17-one 3-sulfate ester to a different pharmaceutically acceptable salt of 3α-hydroxy-5(10)-estrene-17-one 3-sulfate ester or 3β-hydroxy-5(10)-estrene-17-one 3-sulfate ester or
c) converting 3α-hydroxy-5(10)-estrene-17-one or 3β-hydroxy-5(10)-estrene-17-one 23 to a pharmaceutically acceptable salt of 3α-hydroxy-5(10)-estrene-17-one 3-sulfate ester or 3β-hydroxy-5(10)-estrene-17-one 3-sulfate ester.

3α-hydroxy-5(10)-estrene-17-one 3-sulfate ester or 3β-hydroxy-5(10)-estrene-17-one 3-sulfate ester may be converted to a pharmaceutically acceptable salt by neutralising the acid with an appropriate base, e.g. with an alkali metal carbonate, an alkaline earth metal carbonate or a primary, secondary, tertiary or quaternary amine carbonate. Alkali metal or alkaline earth metal salts may be prepared by using the appropriate alkali metal hydride e.g. sodium hydride, potassium hydride or lithium hydride.

A pharmaceutically acceptable salt of 3α-hydroxy-5(10)-estrene-17-one 3-sulfate ester or 3β-hydroxy-5(10)-estrene-17-one 3-sulfate ester may be converted to a different pharmaceutically acceptable salt of 3α-hydroxy-5(10)-estrene-17-one 3-sulfate ester or 3β-hydroxy-5(10)-estrene-17-one 3-sulfate ester by displacement, by using an ion exchange resin or by double decomposition (metastasis). Displacement of a weak base with a stronger one may be utilised to convert, e.g. an amine salt to an alkali metal salt or an alkaline earth metal salt using an appropriate base, e.g. a hydroxide. For example a trialkylamine salt such as a triethylamine salt may be converted to an alkali metal salt such as a sodium salt by treating it with an alkali metal hydroxide such as aqueous sodium hydroxide. The displacement may be carried out using an ion exchange resin. Alternatively one salt may be converted to another by double decomposition, e.g. an alkaline earth metal salt such as the calcium salt may be replaced with an alkali metal salt. E.g. the calcium salt of 3α-hydroxy-5(10)-estrene-17-one 3-sulfate ester or 3β-hydroxy-5(10)-estrene-17-one 3-sulfate ester may be dissolved in water followed by the addition of e.g. sodium carbonate. Insoluble calcium carbonate would then precipitate out to provide the sodium salt of 3α-hydroxy-5(10)-estrene-17-one 3-sulfate ester or 3β-hydroxy-5(10)-estrene-17-one 3-sulfate ester.

A pharmaceutically acceptable salt of 3α-hydroxy-5(10)-estrene-17-one 3-sulfate ester or 3β-hydroxy-5(10)-estrene-17-one 3-sulfate ester may be prepared by directly converting 3α-hydroxy-5(10)-estrene-17-one or 3β-hydroxy-5(10)-estrene-17-one to a pharmaceutically acceptable salt of 3α-hydroxy-5(10)-estrene-17-one 3-sulfate ester or 3β-hydroxy-5(10)-estrene-17-one 3-sulfate ester. This may be performed by reacting it with the appropriate aminesulfurtrioxide complex, e.g. by reacting it with a trialkylaminesulfurtrioxide (such as triethylaminesulfurtrioxide complex) to provide the corresponding trialkylamine salt (such as the triethylamine salt). If desired the salt may then be converted to another salt of the invention as described above. Alkali metal or alkaline earth metal salts may be prepared by treating 3α-hydroxy-5(10)-estrene-17-one with the appropriate alkali metal hydride e.g. sodium hydride, potassium hydride or lithium hydride to produce the corresponding alkoxide *in situ* and then adding a trialkylaminesulfurtrioxide (such as triethylaminesulfurtrioxide complex) to provide the corresponding trialkylamine salt (such as the triethylamine salt). If desired the salt may then be converted to another salt of the invention as described above.

The present invention also provides a pharmaceutically acceptable salt of 3β-hydroxy-5(10)-estrene-17-one 3-sulfate ester, a pharmaceutically acceptable salt of 3α-hydroxy-5(10)-estrene-17-one 3-sulfate ester or a mixture thereof prepared by a chemical process, particularly those prepared according to the processes described above. The invention also provides a pharmaceutically acceptable salt of 3β-hydroxy-5(10)-estrene-17-one 3-sulfate ester, a pharmaceutically acceptable salt of 3α-hydroxy-5(10)-estrene-17-one 3-sulfate ester or a mixture thereof obtainable by such processes.

The compounds of this invention can be prepared from readily available starting materials as shown in Scheme I for the sodium salts of 3β-hydroxy-5(10)-estrene-17-one 3-sulfate ester (**9**) and 3α-hydroxy-5(10)-estrene-17-one 3-sulfate ester (**8**).

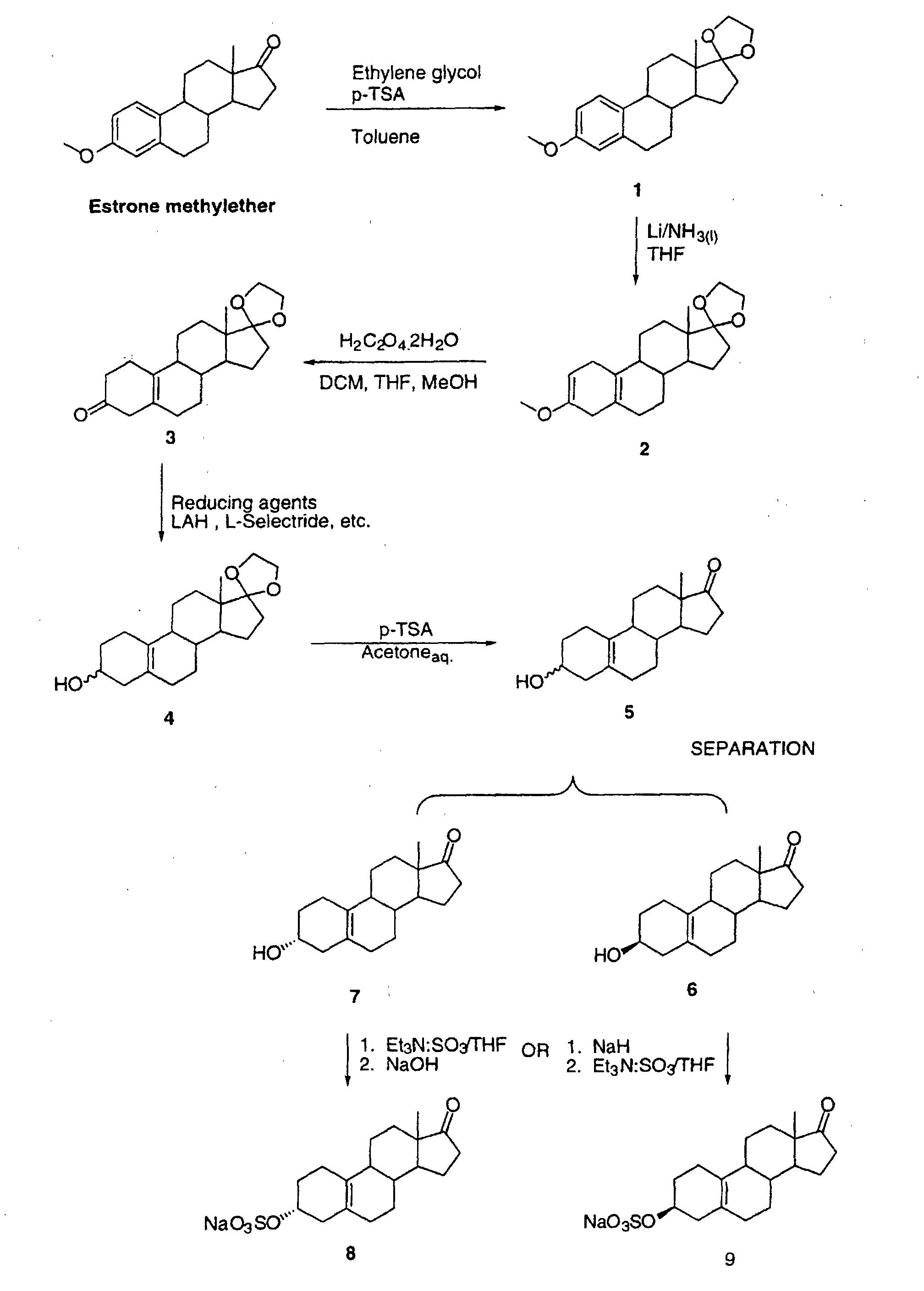

The compounds of this invention are estrogenic and are therefore useful in providing estrogen replacement therapy following ovariectomy or menopause, and in relieving symptoms related to estrogen deficiency, including vasomotor symptoms, such as hot flushes, and other menopausal related conditions, such as vaginal atrophy, vaginitis, and atrophic changes of the lower urinary tract which may cause increased urinary frequency, incontinence, and dysuria. The compounds of this invention are useful in preventing bone loss and in the inhibition or treatment of osteoporosis. The compounds of this invention are cardioprotective and they are useful in the treatment of atherosclerosis, ischemic disease, and hypertension. These cardiovascular protective properties are of great importance when treating postmenopausal patients with estrogens to prevent osteoporosis and in the male when estrogen therapy is indicated. The compounds of this invention are also antioxidants, and are therefore useful in treating or inhibiting free radical induced disease states. Specific situations in which antioxidant therapy is indicated to be warranted are with cancers, central nervous system disorders, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke. Additionally, the compounds of this invention are useful in the suppression of lactation, and in the prophylaxis and treatment of mumps orchitis.

The compounds of this invention can be used alone as a sole therapeutic agent or can be used in combination with other agents, such as other estrogens, progestins, or and androgens.

The compounds of this invention can be formulated neat or with a pharmaceutical carrier for administration, the proportion of which is determined by the solubility and chemical nature of the compound, chosen route of administration and standard pharmacological practice. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, lethicins, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally or vaginally in the form of a conventional suppository. For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

The dosage requirements vary with the particular compositions employed, the route of administration, the severity of the symptoms presented and the particular subject being treated. Based on the results obtained in the standard pharmacological test procedures, projected daily dosages of active compound would be 0.02 µg/kg-750 µg/kg. Treatment will generally be initiated with small dosages less than the optimum dose of the compound. Thereafter the dosage is increased until the optimum effect under the circumstances is reached; precise dosages for oral, parenteral, nasal, or intrabronchial administration will be determined by the administering physician based on experience with the individual subject treated. Preferably, the pharmaceutical composition is in unit dosage form, e.g. as tablets or capsules. In such form, the composition is sub-divided in unit dose containing appropriate quantities of the active ingredient; the unit dosage forms can be packaged compositions, for example, packeted powders, vials, ampoules, prefilled syringes or sachets containing liquids. The unit dosage form can be, for example, a capsule or tablet itself, or it can be the appropriate number of any such compositions in package form.

## Claims

1. A compound which is a pharmaceutically acceptable salt of 3β-hydroxy-5(10)-estrene-17-one 3-sulfate ester.

2. The compound of claim 1 wherein the pharmaceutically acceptable salt of the 3-sulfate ester is an alkali metal salt, alkaline earth metal salt,. ammonium salt, alkylammonium salt containing 1-6 carbon atoms, dialkylammonium salt containing 1-6 carbon atoms in each alkyl group, trialkylammonium salt containing 1-6 carbon atoms in each alkyl group or tetraalkylammonium salt containing 1-6 carbon atoms in each alkyl group.

3. 3β-Hydroxy-5(10)-estrene-17-one 3-sulfate ester sodium salt.

4. A compound which is a pharmaceutically acceptable salt of 3α-hydroxy-5(10)-estrene-17-one 3-sulfate ester.

5. The compound of claim 4 wherein the pharmaceutically acceptable salt of the 3-sulfate ester is an alkali metal salt, alkaline earth metal salt, ammonium salt, alkylammonium salt containing 1-6 carbon atoms, dialkylammonium salt containing 1-6 carbon atoms in each alkyl group, trialkylammonium salt containing 1-6 carbon atoms in each alkyl group or tetraalkylammonium salt containing 1-6 carbon atoms in each alkyl group.

6. A pharmaceutical composition which comprises a pharmaceutically acceptable salt of 3β-hydroxy-5(10)-estrene-17-one 3-sulfate ester and a pharmaceutical carrier.

7. A pharmaceutical composition which comprises a pharmaceutically acceptable salt of 3α-hydroxy-5(10)-estrene-17-one 3-sulfate ester and a pharmaceutical carrier.

8. A pharmaceutical composition which comprises at least 1% of a compound as defined in any one of claims 1 to 5.

9. A compound as defined in any one of claims 1 to 5 for use as a medicament.

10. Use of a compound as defined in any one of claims 1 to 5 in the preparation of a medicament for:
a) inhibiting or treating free radical induced disease states,
b) inhibiting endogenous free radical involvement in disease development of cancers, central nervous system disorders, dementias, Alzheimer's disease, bone disease, aging, inflammatory disorders, peripheral vascular disease, rheumatoid arthritis, autoimmune diseases, respiratory distress, emphysema, prevention of reperfusion injury, viral hepatitis, chronic active hepatitis, tuberculosis, psoriasis, systemic lupus erythematosus, adult respiratory distress syndrome, central nervous system trauma and stroke, or injury during reperfusion procedures,
c) providing estrogen replacement therapy or treating estrogen deficiency,
d) treating vasomotor symptoms related to estrogen deficiency,
e) treating or inhibiting osteoporosis or
f) treating or inhibiting atherosclerosis
in a mammal.

11. A process for the preparation of a pharmaceutically acceptable salt of 3α-hydroxy-5(10)-estrene-17-one 3-sulfate ester or a pharmaceutically acceptable salt of 3β-hydroxy-5(10)-estrene-17-one 3-sulfate ester which comprises:
a) converting 3α-hydroxy-5(10)-estrene-17-one 3-sulfate ester or 3β-hydroxy-5(10)-estrene-17-one 3-sulfate ester to a pharmaceutically acceptable salt or
b) converting a pharmaceutically acceptable salt of 3α-hydroxy-5(10)-estrene-17-one 3-sulfate ester or 3β-hydroxy-5(10)-estrene-17-one 3-sulfate ester or to a different pharmaceutically acceptable salt of 3α-hydroxy-5(10)-estrene-17-one 3-sulfate ester or 3β-hydroxy-5(10)-estrene-17-one 3-sulfate ester or
c) converting 3α-hydroxy-5(10)-estrene-17-one or 3β-hydroxy-5(10)-estrene-17-one 23 to a pharmaceutically acceptable salt of 3α-hydroxy-5(10)-estrene-17-one 3-sulfate ester or 3β-hydroxy-5(10)-estrene-17-one 3-sulfate ester.

## Patentansprüche

1. Verbindung, welche ein pharmazeutisch annehmbares Salz von 3β-Hydroxy-5(10)-estren-17-on-3-sulfatester ist.

2. Verbindung nach Anspruch 1, worin das pharmazeutisch annehmbare Salz des 3-Sulfatesters ein Alkalimetallsalz, Erdalkalimetallsalz, Ammoniumsalz, Alkylammoniumsalz, welches 1-6 Kohlenstoffatome enthält, Dialkylammoniumsalz, welches 1-6 Kohlenstoffatome in jeder Alkylgruppe enthält, Trialkylammoniumsalz, welches 1-6 Kohlenstoffatome in jeder Alkylgruppe enthält, oder Tetraalkylammoniumsalz, welches 1-6 Kohlenstoffatome in jeder Alkylgruppe enthält, ist.

3. 3β-Hydroxy-5(10)-estren-17-on-3-sulfatester-natriumsalz.

4. Verbindung, welche ein pharmazeutisch annehmbares Salz von 3α-Hydroxy-5(10)-estren-17-on-3-sulfatester ist.

5. Verbindung nach Anspruch 4, worin das pharmazeutisch annehmbare Salz des 3-Sulfatesters ein Alkalimetallsalz, Erdalkalimetallsalz, Ammoniumsalz, Alkylammoniumsalz, welches 1-6 Kohlenstoffatome enthält, Dialkylammoniumsalz, welches 1-6 Kohlenstoffatome in jeder Alkylgruppe enthält, Trialkylammoniumsalz, welches 1-6 Kohlenstoffatome in jeder Alkylgruppe enthält, oder Tetraalkylammoniumsalz, welches 1-6 Kohlenstoffatome in jeder Alkylgruppe enthält, ist.

6. Pharmazeutische Zusammensetzung, welche ein pharmazeutisch annehmbares Salz von 3β-Hydroxy-5(10)-estren-17-on-3-sulfatester und einen pharmazeutischen Träger umfasst.

7. Pharmazeutische Zusammensetzung, welche ein pharmazeutisch annehmbares Salz von 3α-Hydroxy-5(10)-estren-17-on-3-sulfatester und einen pharmazeutischen Träger umfasst.

8. Pharmazeutische Zusammensetzung, welche mindestens 1% einer Verbindung, wie in einem der Ansprüche 1 bis 5 definiert, umfasst.

9. Verbindung, wie in einem der Ansprüche 1 bis 5 definiert, zur Verwendung als Medikament.

10. Verwendung einer Verbindung, wie in einem der Ansprüche 1 bis 5 definiert, bei der Herstellung eines Medikaments zum:
a) Hemmen oder Behandeln von durch freie Radikale verursachten Krankheitszuständen
b) Hemmen endogener Einbeziehung von freien Radikalen in die Krankheitsentwicklung von Krebsen, Störungen des zentralen Nervensystems, Demenzen, Alzheimererkrankung, Knochenerkrankung, Alterung, Entzündungsstörungen, peripher-vaskulärer Krankheit, Rheumatoidarthritis, Autoimmunerkrankung, Atemnot, Emphysem, Verhinderung von Reperfusionsschaden, viraler Hepatitis, chronisch aktiver Hepatitis, Tuberkulose, Psoriasis, systemischem Lupus erythematodes, Atemnotsyndrom des Erwachsenen, Trauma und Schlaganfall des zentralen Nervensystems oder Schäden während Reperfusionsverfahren,
c) Vorsehen von östrogen-Ersatztherapie oder Behandeln von Östrogenmangel,
d) Behandeln von vasomotorischen Symptomen in Verbindung mit Östrogenmangel,
e) Behandeln oder Hemmen von Osteoporose oder
f) Behandeln oder Hemmen von Atherosklerose
in einem Säuger.

11. Verfahren für die Herstellung eines pharmazeutisch annehmbaren Salzes von 3α-Hydroxy-5(10)-estren-17-on-3-sulfatester oder eines pharmazeutisch annehmbaren Salzes von 3β-Hydroxy-5(10)-estren-17-on-3-sulfatester, welches umfasst:
a) Umwandeln von 3α-Hydroxy-5(10)-estren-17-on-3-sulfatester oder 3β-Hydroxy-5(10)-estren-17-on-3-sulfatester in ein pharmazeutisch annehmbares Salz oder
b) Umwandeln eines pharmazeutisch annehmbaren Salzes von 3α-Hydroxy-5(10)-estren-17-on-3-sulfatester oder 3β-Hydroxy-5(10)-estren-17-on-3-sulfatester in ein anderes pharmazeutisch annehmbares Salz von 3α-Hydroxy-5(10)-estren-17-on-3-sulfatester oder 3β-Hydroxy-5(10)-estren-17-on-3-sulfatester oder
c) Umwandeln von 3α-Hydroxy-5(10)-estren-17-on oder 3β-Hydroxy-5(10)-estren-17-on 23 in ein pharmazeutisch annehmbares Salz von 3α-Hydroxy-5(10)-estren-17-on-3-sulfatester oder 3β-Hydroxy-5(10)-estren-17-on-3-sulfatester.

## Revendications

1. Composé qui est un sel pharmaceutiquement acceptable d'ester 3-sulfate de 3β-hydroxy-5(10)-oestrène-17-one.

2. Composé suivant la revendication 1, dans lequel le sel pharmaceutiquement acceptable d'ester 3-sulfate est un sel de métal alcalin, un sel de métal alcalino-terreux, un sel d'ammonium, un sel d'alkylammonium contenant 1-6 atomes de carbone, un sel de dialkylammonium contenant 1-6 atomes de carbone dans chaque groupement alkyle, un sel de trialkylammonium contenant 1-6 atomes de carbone dans chaque groupement alkyle ou un sel de tétraalkylammonium contenant 1-6 atomes de carbone dans chaque groupement alkyle.

3. Sel de sodium d'ester 3-sulfate de 3β-hydroxy-5(10)-oestrène-17-one.

4. Composé qui est un sel pharmaceutiquement acceptable d'ester 3-sulfate de 3α-hydroxy-5(10)-oestrène-17-one.

5. Composé suivant la revendication 4, dans lequel le sel pharmaceutiquement acceptable d'ester 3-sulfate est un sel de métal alcalin, un sel de métal alcalino-terreux, un sel d'ammonium, un sel d'alkylammonium contenant 1-6 atomes de carbone, un sel de dialkylammonium contenant 1-6 atomes de carbone dans chaque groupement alkyle, un sel de trialkylammonium contenant 1-6 atomes de carbone dans chaque groupement alkyle ou un sel de tétraalkylammonium contenant 1-6 atomes de carbone dans chaque groupement alkyle.

6. Composition pharmaceutique qui comprend un sel pharmaceutiquement acceptable d'ester 3-sulfate de 3β-hydroxy-5(10)-oestrène-17-one et un vecteur pharmaceutique.

7. Composition pharmaceutique qui comprend un sel pharmaceutiquement acceptable d'ester 3-sulfate de 3α-hydroxy-5(10)-oestrène-17-one et un vecteur pharmaceutique.

8. Composition pharmaceutique qui comprend au moins 1% d'un composé comme défini dans l'une quelconque des revendications 1 à 5.

9. Composé comme défini dans l'une quelconque des revendications 1 à 5, pour une utilisation comme médicament.

10. Utilisation d'un composé comme défini dans l'une quelconque des revendications 1 à 5, dans la préparation d'un médicament pour :
a) inhiber ou traiter des états de maladie induits par des radicaux libres.
b) inhiber une implication de radicaux libres endogènes dans le développement pathologique de cancers, de troubles du système nerveux central, de démences, de la maladie d'Alzheimer, d'une maladie osseuse, du vieillissement, de troubles inflammatoires, d'une maladie vasculaire périphérique, d'une arthrite rhumatoïde, de maladies auto-immunes, d'une détresse respiratoire, d'un emphysème, d'une prévention de lésion de reperfusion, d'une hépatite virale, d'une hépatite chronique active, de la tuberculose, du psoriasis, du lupus érythémateux disséminé, d'un syndrome de détresse respiratoire de l'adulte, d'un traumatisme au système nerveux central et d'un ictus, ou d'une lésion pendant des procédures de reperfusion,
c) procurer une thérapie de substitution aux oestrogènes ou le traitement d'une carence en oestrogènes,
d) traiter des symptômes vasomoteurs associés à une déficience en oestrogènes,
e) traiter ou inhiber une ostéoporose ou
f) traiter ou inhiber une athérosclérose
chez un mammifère.

11. Procédé pour la préparation d'un sel pharmaceutiquement acceptable d'ester 3-sulfate de 3α-hydroxy-5(10)-oestrène-17-one ou d'un sel pharmaceutiquement acceptable d'ester 3-sulfate de 3β-hydroxy-5(10)-oestrène-17-one qui comprend :
a) la conversion de l'ester 3-sulfate de 3α-hydroxy-5(10)-oestrène-17-one ou de l'ester 3-sulfate de 3β-hydroxy-5(10)-oestrène-17-one en un sel pharmaceutiquement acceptable, ou
b) la conversion d'un sel pharmaceutiquement acceptable d'ester 3-sulfate de 3α-hydroxy-5(10)-oestrène-17-one ou d'ester 3-sulfate de 3β-hydroxy-5(10)-oestrène-17-one en un sel pharmaceutiquement acceptable différent d'ester 3-sulfate de 3α-hydroxy-5(10)-oestrène-17-one ou d'ester 3-sulfate de 3β-hydroxy-5(10)-oestrène-17-one ou
c) la conversion de la 3α-hydroxy-5(10)-oestrène-17-one ou de la 3β-hydroxy-5(10)-oestrène-17-one 23 en un sel pharmaceutiquement acceptable d'ester 3-sulfate de 3α-hydroxy-5(10)-oestrène-17-one ou d'ester 3-sulfate de 3β-hydroxy-5(10)-oestrène-17-one.
